# EUROPEAN PATENT SPECIFICATION

(11) **EP 1 941 852 B1**
(45) Date of publication and mention of the grant of the patent: **04.07.2012**
(21) Application number: 07024333.2
(22) Date of filing: 14.12.2007
(51) Int. Cl.: A61F 13/15

(54) **Individually packaged disposable absorbent article**
Einzeln verpackter, absorbierender Wegwerfartikel
Article absorbant pouvant être disposé en emballage individuel

(30) Priority: 21.12.2006 EP 06026546
(43) Date of publication of application: 09.07.2008
(73) Proprietor: The Procter & Gamble Company, Cincinnati, OH 45202 (US)
(72) Inventor: Toro, Carlo, 65012 Cepagatti (Pescara) (IT); Salone, Fiorello, 65124 Pescara (IT); Massacesi, Ettore, 65015 Montesilvano (Pescara) (IT); Fuhrmann, Jan, 60316 Frankfurt (DE); Haesler, Peter Ludwig, 65510 Idstein (DE); Schmidt, Horst, 65929 Frankfurt (DE); Pompei, Enzo, 65120 Pescara (IT); Partenza, Vincenzo, 65010 Elice (Pescara) (IT)
(74) Representative: Briatore, Andrea

(56) References cited:
- EP-A- 0 737 731
- EP-A1- 0 652 102
- WO-A-03/030796
- US-A- 5 972 473
- US-A1- 2005 137 555
- US-A1- 2005 288 644

## Description

### FIELD OF THE INVENTION

The present invention relates to disposable absorbent articles, for example sanitary napkins and the like, which are individually packaged prior to use.

### BACKGROUND OF THE INVENTION

Disposable absorbent articles of personal hygiene are known in the art. Typical examples include sanitary napkins, panty liners, adult incontinence articles, infant diapers, paper towels, bath tissue and facial tissue. Such articles are often used to absorb and retain bodily fluids and other exudates excreted by the human body. Many disposable absorbent articles have the same basic structure: an absorbent core encased between a liquid permeable, user contacting topsheet, which permits liquid to penetrate its thickness and contact the absorbent core where liquid is retained, and a backsheet, which may be liquid impermeable.

While there are a great many variations in the specific structural features of disposable absorbent articles, they are typically presented to consumers in the same manner. Essentially, the disposable absorbent article, irrespective of the specific structural features used, is packaged in a box or bag from which the consumer withdraws the article, as needed. In order to protect the article from soiling or contamination from the time it is removed from the box or bag until the article is used, for example if a woman wanted to carry a sanitary napkin with her for use away from home, the articles may be individually packaged within the box or bag by means of a sheet of material which is wrapped around the individual article.

A typical individual package for disposable absorbent articles is disclosed, for example, in US patent 4,556,146, which describes a disposable absorbent article, typically a sanitary napkin, associated with a wrapper which overlays one major surface of the sanitary napkin. The wrapper extends beyond the perimeter of the disposable absorbent article so that when the disposable absorbent article and the wrapper are folded as a unit, the longitudinal side flaps of the wrapper, which extend beyond the longitudinal sides of the article, may be frangibly sealed thereby providing the disposable absorbent article with an individual package. It is also common to provide the disposable absorbent article with an adhesive element on the garment facing side of the backsheet which, in use, serves to affix the absorbent article to the wearer's undergarment thereby maintaining the absorbent article in place against the wearer's body. The adhesive element may take the form of a coating of adhesive which is in strips or other suitable pattern. For example, the garment facing side of the backsheet can be coated uniformly with a layer of pressure sensitive hot melt adhesive. The wrapper overlays the garment facing side of the backsheet with the longitudinal flap portions extending beyond the longitudinal perimeter segments of the absorbent article. The wrapper typically is not folded onto or otherwise brought into contact with the body facing side of the topsheet; in other words, the surface of the wrapper facing the garment facing side of the backsheet is in face to face relation substantially with said side only of the backsheet. The wrapper is typically releasably affixed to the disposable absorbent article, e.g. a sanitary napkin, by the aforementioned adhesive element. When an adhesive element is used in this manner, it is not necessary to provide the absorbent articles with a separate release sheet in order to protect the adhesive element before use, as this function is provided by the wrapper.

To individually package the absorbent article, the article and the affixed wrapper can be typically folded as a unit. That is, they are folded together with the wrapper remaining in place with respect to the absorbent article. Typically, the absorbent article is folded lengthwise into thirds about two fold axes. The longitudinal side flaps or flap portions of the wrapper are frangibly sealed using any of the well known sealing techniques. For example, the longitudinal flap portions may be heat sealed, glued, ultrasonically bonded, or crimped.

In use, the individually packaged absorbent article is provided to a user. The user may then break the frangible seals, unfold the wrapper/absorbent article unit and separate the wrapper from the absorbent article, for example a sanitary napkin, exposing the adhesive element. The absorbent article may then be used as such devices normally are, typically being adhered by means of the adhesive element to the crotch portion of an undergarment, which is subsequently worn.

An advantage of an individually packaged disposable absorbent article, for example a sanitary napkin or a pantiliner, is discreetness, as a user does not need to take with her an entire box or bag of products, but only a small number of individually packaged articles, as needed for subsequent use, for example when staying away from home. However, materials commonly used for the wrapper, typically flexible materials which can be also liquid impermeable such as polymeric films, for example polyethylene films, can be rather noisy when the packaged product is manipulated in order to unfold the wrapper with the absorbent article, breaking the seals along the longitudinal flap portions of the wrapper, and then separating the article from the wrapper itself by detaching the adhesive element. This is typically due to the nature of the wrapper material, namely its composition, and to its relatively high thickness, usually around 20-30 µ, for example 25 µ, which is dictated by process reasons. In a high speed production line the material of the wrapper is typically provided in a continuous web; the adhesive element may be provided in selected areas directly onto the wrapper material continuous web, and then absorbent articles are provided onto each respective adhesive element, by adhering thereon the garment facing side of the backsheet. The wrapper material continuous web may be folded together with the applied absorbent article, and cut and sealed in order to form individually packaged articles. When the user, after unfolding the package and breaking the seals, separates the absorbent article from the wrapper material, at least some of the adhesive element remain on the garment facing surface of the backsheet since the respective surface of the wrapper element is typically provided with means having an inferior adhesion to the adhesive material. For example, the wrapper may include a coating of a suitable low adhesion material, such as a silicone coating.

The wrapper material may be subjected to substantial stress in known high speed processes to produce individually packaged absorbent articles, as described above. The stress can be both mechanical and thermal, as the adhesive material, typically a hot melt adhesive, is provided onto the wrapper material in molten state, hence at relatively high temperature.

US 2005/0288644 and US 2005/0137555 generically describe individually packaged absorbent articles, typically interlabial devices. WO 93/030796, EP 737731, EP 652102 and its equivalent US 5972473 describe different materials which can be used as packaging materials, for example for sanitary articles or also food articles.

A simple reduction of the wrapper material calliper, which could reduce the noisiness of the material upon manipulation, may also reduce its resistance to the stresses normally encountered during the manufacturing process. This could result in the material breaking or deforming, hence impairing the production process and the quality of the resulting products, or leading to a lower production line speed.

It is therefore desirable to provide an individually packaged disposable absorbent article which is more discreet, particularly less noisy upon use, than known products. It would also be desirable to provide such an article without compromising process conditions, such as, for example, the line speed.

### SUMMARY OF THE INVENTION

The present invention addresses the above need by providing an individually packaged absorbent article according to claim 1.

### BRIEF DESCRIPTION OF THE DRAWINGS

Figure 1 is a partially cut away perspective view of an absorbent article and its associated wrapper prior to being folded and sealed.

Figure 2 is a partially cut away perspective view of an absorbent article and its associated wrapper after they have been folded and sealed to form an individually packaged absorbent article.

### DETAILED DESCRIPTION OF THE INVENTION

Referring now to the drawings there is shown an individually packaged disposable absorbent article embodying the teachings of the present invention. As used herein the term "absorbent article" refers to those articles intended to absorb and retain liquid and in particular to those articles which are placed against or in proximity to a wearer's body to absorb and contain the various liquids discharged from the body (e.g. blood, menses, urine). A "disposable absorbent article" is an absorbent article which is intended to be discarded after a single use (i.e., they are not intended to be laundered or otherwise restored and reused). Typical disposable absorbent articles according to the present invention can be absorbent articles for feminine hygiene such as sanitary napkins and panty liners, commonly referred to collectively as catamenial pads, light incontinence products, or the like.

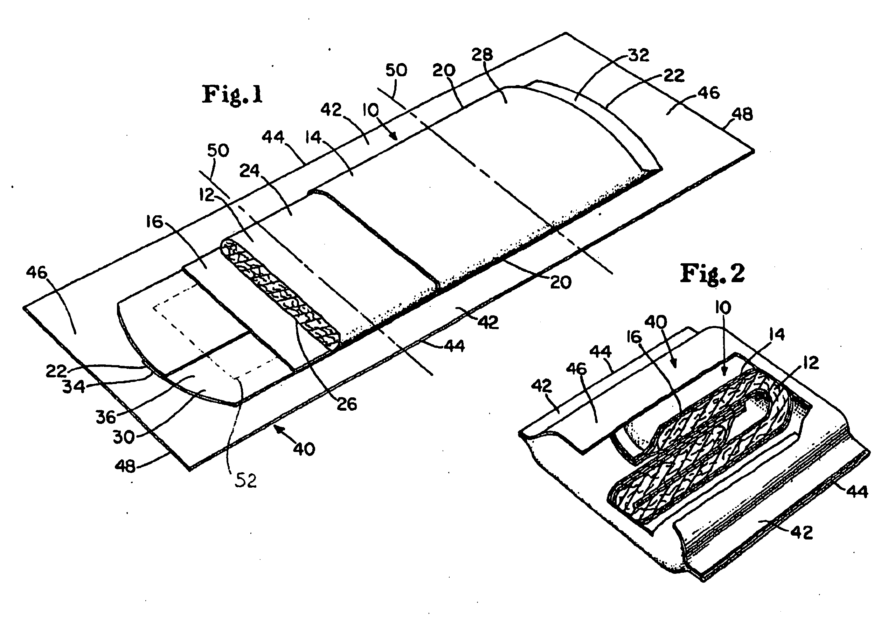

FIG. 1 is a partially cut away perspective view of a catamenial pad 10 and a wrapper 40 prior to being folded and sealed as set forth in greater detail herein below. A catamenial pad is a disposable absorbent article which is worn by females external to and in the proximity of the urogenital region and which is intended to absorb and contain menstrual fluids and other vaginal discharges. As used herein the term "catamenial pad" includes pantiliners which are often worn by females external to the urogenital region between periods of heavy menstrual flow and which are intended to absorb light menstrual flow and nonmenstrual vaginal discharges. The primary difference between catamenial pads used during periods of heavy menstrual flow and catamenial pads used between periods of heavy menstrual flow (i.e., pantiliners) is the absorbent capacity of the pad.

While the present invention will be described with reference to a catamenial pad, it should be understood that the present invention has application in the context of other disposable absorbent articles such as, for example, light incontinence products. Further, the teachings of this invention have application to catamenial pads manufactured according to the teachings of any of the multitudinous references in the catamenial pad art. A typical catamenial pad embodiment will now be described briefly.

As can be seen in FIG. 1, the catamenial pad 10 basically comprises an absorbent pad 12 an envelope sheet 14 and a barrier 16. The catamenial pad 10 has a perimeter generally comprising longitudinal pcrimctcr segments, or longitudinal sides 20 and transverse perimeter segments, or transverse ends 22. The perimeter defines the outer boundary of the catamenial pad 10, while the longitudinal perimeter segments 20 and the transverse perimeter segments 22 define the outer boundary of the catamenial pad 10 along each longitudinal side and each transverse end respectively.

The absorbent pad 12 is generally compressible, conformable, and non-irritating to the user's skin and may be manufactured from a wide variety of absorbent materials which are capable of absorbing and retaining liquids. For example, a batt of absorbent fibers, a multiplicity of plies of creped cellulose wadding, or any equivalent material may be used. The absorbent capacity of the material used, however, must be sufficient to absorb and retain the expected liquid loading in the intended use of the absorbent article without undue bulk. An example of a suitable catamenial pad 10 intended to receive heavy menstrual discharges of approximately 40 millilitres, may include about 8 grams of comminuted wood pulp, generally referred to as airfelt.

The shape and dimensions of the absorbent pad 12 are selected to permit the disposable absorbent article to conform to and fit about the portion of the body against which it will be placed. Often, as in the embodiment illustrated, the general shape and dimensions of the catamenial pad 10 will be determined by the shape and dimensions of the absorbent pad 12. In the embodiment illustrated in the figures, the shape and dimensions of the absorbent pad 12 were selected to permit the catamenial pad 10 to conform to the urogenital region of the wearer's body. While the shape and dimensions of the absorbent pad 12 may be varied, it has been found that a generally planar configuration having a first major surface, or body facing side, 24 and a second major surface, or garment facing side, 26 is suitable. The first major surface 24 is that surface of the absorbent pad 12 facing toward the source of liquid (i.e. toward the wearer's body) and the second major surface 26 is that surface of the absorbent pad 12 facing away from the source of liquid. An example of a suitable catamenial pad 10 has a generally rectangular, planar shaped absorbent pad 12 having a length of about 9.0 inches (22.9 centimeters) and a width of about 2.5 inches (6.4 centimeters). It should be understood, however, that other shapes (e.g. elongated ovals, triangles, squares, etc.) and other dimensions may he used.

The catamenial pad 10 may include an envelope sheet 14 that encases the absorbent pad 12 and is preferably compliant, soft feeling, and non-irritating to the wearer's body. The envelope sheet 14 can help maintain the structural integrity of the absorbent pad 12 and has a first and a second end flap 30 and 32 respectively. The envelope sheet 14 may be wrapped about the absorbent pad 12 and may be affixed to itself along a seam 34 which is adjacent the second major surface 26 and which traverses the catamenial pad 10 longitudinally. The first and second flaps 30 and 32 respectively extend beyond the transverse ends of the absorbent pad 12 and are typically sealed so as to completely encase the absorbent pad 12 within the envelope sheet 14. The portion of the envelope sheet 14 overlaying the first major surface 24 is the topsheet portion 28 corresponding to the body facing side of the article, and the portion of the envelope sheet 14 overlaying the second major surface 26 is the backsheet portion 36 of the envelope sheet 14, corresponding to the garment facing side of the article. The topsheet portion 28 is liquid permeable. In use the topsheet portion 28 may contact the skin of the catamenial pad wearer and permit the transmission of liquid through its thickness to the absorbent pad 12 where the liquid may be retained.

There are many suitable materials from which the envelope sheet 14 may be manufactured. The topsheet portion 28 may be manufactured from either hydrophobic or hydrophilic fibers and may, for example, be carded, spun bonded, melt blown, or air laid. Alternatively, the topsheet portion 28 may be a continuous film or sheet of, for example, thermoplastic material which is apertured. A suitable topsheet portion 28 is described in U.S. Pat. No. 4,324,246 which issued to Mullane et al on Apr. 13, 1982.

The topsheet portion 28 and the backsheet portion 36 may either be integral (i.e. the backsheet portion 36 and the topsheet portion 28 are separate elements affixed to each other) or unitary (i.e. the backsheet portion 36 and the topsheet portion 28 are formed from a continuous and undivided sheet of material) and may either have the same or different physical properties. The embodiment of FIG. 1 shows the topsheet portion 28 and the backsheet portion 36 as being unitary.

To help prevent liquids absorbed by the absorbent pad 12 from penetrating through the backsheet portion 36, it may be advantageous to interpose a barrier 16 at the interface between the second major surface 26 of the absorbent pad 12 and the backsheet portion 36. The barrier 16 may be manufactured from any flexible, liquid impermeable material which is non-irritating to the wearer. In certain embodiments, the barrier 16 may be a sheet of polyethylene which is coincident with the backsheet portion 36.

Alternatively, the envelope sheet 14 may comprise a topsheet portion 28 and a backsheet portion 36 which are made integral with each other by affixing them together about their periphery. In such embodiments, the topsheet portion 28 may be at least partially liquid impervious and the backsheet portion 36 may be liquid pervious or wholly or partially liquid impervious.

It is common to provide the catamenial pad 10 with an adhesive element 52, partially shown in dotted line in FIG. 1. The adhesive element 52 is positioned on the garment facing side of the article, namely on the backsheet portion 36 and, in use, serves to affix the catamenial pad 10 to the wearer's undergarments thereby maintaining the catamenial pad 10 in place against the wearer's body. The adhesive element 52 may take the form of a coating of adhesive which is in strips or any other suitable pattern. The backsheet portion 36 may be coated uniformly with a layer of a pressure sensitive hot melt adhesive such as, for example, NS34-2823 as manufactured by National Starch and Chemical of Bridgewater, N.J.

In accordance with the teachings of this invention, a wrapper 40 is associated with, and typically can have dimensions generally larger than those of the catamenial pad 10. Thus, the wrapper 40 has longitudinal flap portions 42 comprising that portion of the wrapper 40 between the longitudinal edge 44 of the wrapper and the longitudinal perimeter segment or longitudinal side 20 of the catamenial pad 10. In the embodiment illustrated in FIG. 1, the wrapper 40 also has transverse flap portions 46 comprising that portion of the wrapper 40 between the transverse edges 48 of the wrapper 40 and the transverse perimeter segments or transverse ends 22 of the catamenial pad 10.

The wrapper 40 is shown overlaying the garment facing side of the absorbent article, namely the backsheet portion 36, with the longitudinal flap portions 42 typically extending beyond the longitudinal perimeter segments 20. In the absorbent shown in FIG. 1, the wrapper 40 is not folded onto or otherwise brought into contact with the topsheet portion 28. In other words, the surface of the wrapper 40 facing the backsheet portion 36 is in face to face relation with the backsheet portion 36 only. The wrapper 40 is releasably affixed to the catamenial pad 10 by the aforementioned adhesive element 52, hence it is also defined as a releasable wrapper 40 according to the present invention. If an adhesive element is used in this manner, it is not necessary to provide the absorbent article with a separate release sheet as is commonly done in prior art devices, as the function of protecting the adhesive element from contamination prior to use is provided by the wrapper.

To individually package the catamenial pad 10, the catamenial pad 10 and the affixed wrapper 40 are folded as a unit. That is, they are folded together with the wrapper 40 remaining in place with respect to the catamenial pad 10. According to the present invention, the absorbent article and the wrapper can be folded as a unit about at least one fold-axis, e.g. a longitudinal or a transverse fold-axis, in order to define a packaged absorbent article. In certain embodiments, the catamenial pad 10 may be folded lengthwise into thirds about two transverse fold-axes 50, as shown in FIG. 2, to define a package comprising the absorbent article. The longitudinal flap portions 42 can be frangibly scaled using any of the well-known scaling techniques. For example, the longitudinal flap portions 42 may be heat sealed, glued, ultrasonically bonded, or crimped.

In use, the individually packaged catamenial pad is provided to a user. The user may then break the seals, unfold the catamenial pad 10, and separate the wrapper 40 from the catamenial pad 10. The catamenial pad 10 may then be used as such devices normally are.

According to the present invention, the releasable wrapper 40 comprises a flexible sheet material having a basis weight of 5-19 g/m², or of 7-18 g/m², or also of 10-16 g/m², and a maximum tensile strength of at least 6.5 N/cm, or of at least 7 N/cm, or also of at least 9 N/cm. The tensile strength is measured according to the ASTM D 882 Standard Test Method for Tensile Properties of Thin Plastic Sheeting, slightly modified as explained in the "Test Method" section below. As known, the tensile strength of flexible sheet materials, for example typically polymeric films, is usually not uniform in all directions, wherein the material is said anisotropic. Typically it has a higher value in the direction corresponding to the direction along which the material has been originally produced, known as machine direction (MD), and has a lower value in the direction generally orthogonal to the machine direction, known as cross direction (CD). The flexible sheet material used for the wrapper 40 of the present invention has hence a maximum tensile strength, and a minimum tensile strength, which generally correspond to the tensile strength in machine direction and cross direction respectively of the flexible sheet material itself. In an already formed wrapper 40, which as shown in FIG. 1 in its open position laid on a flat surface having a typically rectangular shape, the tensile strength can be measured along the directions parallel to the two axes of the rectangle, or more in general to a direction parallel to the major axis of the wrapper, and to a second direction perpendicular thcrcto, typically parallel to the minor axis of the wrapper, in order to identify a maximum and a minimum tensile strength for the material, typically corresponding to the MD and to the CD tensile strength of the original flexible sheet material of the wrapper.

In an embodiment of the present invention the flexible sheet material of the wrapper 40 can also have a minimum tensile strength of at least 3.5 N/cm, or of at least 3.7 N/cm, or also of at least 4 N/cm.

Alternatively, flexible sheet materials having a substantially uniform tensile strength in all directions, also known as isotropic materials, can be used for the wrapper according to the present invention. In such a case the required maximum tensile strength corresponds to the generic, uniform tensile strength of the flexible sheet material.

The wrapper material, having the selected combination of low basis weight, which translates into a low thickness, and high tensile strength, can provide the desired characteristics of low noisiness upon manipulation, and the necessary resistance to overcome known high speed production processes with no disruptions in the process, and without compromising in the quality of the final product.

According to an embodiment of the present invention, the flexible sheet material for the wrapper 40 can be selected among polymeric films having a thickness of 6-20 µ, or of 8-19 µ, or also of 11-17 µ.

The flexible sheet materials of the wrapper 40 according to the present invention also have a good thermal resistance, despite their very low basis weight and caliper, which is advantageous in that they effectively withstand application of known hot melt adhesives in molten state in order to provide the adhesive element in the individually packaged absorbent article of the present invention.

Known means having a reduced adhesion to the adhesive material can be provided to the flexible sheet material for the wrapper 40 of the present invention, namely on the surface which is meant to contact, and be affixed to, the adhesive element on the garment facing surface of the absorbent article. For example a coating with a suitable low adhesion material, typically a silicone coating, may be provided.

According to a further embodiment of the present invention, the wrapper material may be selected in order to have a relatively high coefficient of friction, typically on the surface opposite to that subjected to the low adhesion treatment, hence typically on the surface without silicone coating. A sufficiently high coefficient of friction of the wrapper material may be desirable in high speed manufacturing processes, as it may help prevent that the very thin and light material, both when still in the form of a continuous material web, and particularly after it is already in the form of single bags, from slipping during processing, and can add to its stability. The coefficient of friction of the wrapper material can be evaluated with the standard test method ASTM D 1894-06, as described in details in the "Test Methods" section below. According to this embodiment of the present invention, the coefficient of friction of the wrapper material, typically onto the surface opposite to that interested by the low adhesion treatment (e.g. a silicone coating) is comprised between 0.1 and 0.7, or between 0.2 and 0.6, or even also between 0.3 and 0.5.

According to a still further embodiment of the present invention, the wrapper material may be selected in order to effectively withstand the scaling process performed along the longitudinal flap portions 42 to provide the frangible seals typically torn by the user when opening the package in order to access the pad. Suitable sealing processes, as already mentioned, can comprise gluing, but also, and more typically, mechanical and/or thermal processes, such as ultrasonic bonding and particularly crimping. Such processes involving mechanical and/or thermal treatment of the wrapper material can be particularly stressing and might lead in some cases to tearing or breaking of the wrapper material, particularly when having the low basis weight according to the present invention. According to this embodiment of the present invention the wrapper material can be selected such that it has a tear propagation resistance of at least 1.8 N, or of at least 0.20 N, or of at least 0.25 N. The tear propagation resistance of the wrapper materials according to the present invention can be typically less than 2 N, or less than 1.5 N, or less than 1 N. The tear propagation resistance of the wrapper material can be evaluated according to the standard test method ASTM D 1938-06, slightly modified as explained in the "Test Methods" section below.

Single layer or multilayer polymeric films can be selected for the wrapper material according to the present invention, which are typically obtainable for example through a single layer extrusion or a multilayer co-extrusion process. As known, the only layer, or each layer of the polymeric film can comprise a single polymer or a blend of different polymers.

According to an embodiment of the present invention, the polymeric film for the sheet material of the wrapper 40 may be a polyethylene film comprising at least 0.5% by weight polypropylene, or at least 3% by weight polypropylene. It has been discovered that films made of such polyethylene/polypropylene blends provide the required tensile strength with a surprisingly low basis weight and thickness, and hence are suitable as wrapper materials for the individually packaged absorbent article of the present invention. Typically, such polymeric films can comprise less than 30% by weight, or also less than 15% by weight.

Also, polymeric films suitable as sheet material for the wrapper material according to the present invention can be provided in order to have the selected characteristics in terms of low thickness combined with desired mechanical properties, such as tensile strength or tear propagation resistance, or also coefficient of friction, as explained above, by suitably acting on compositional and/or process parameters. For example polymer average molecular weight, density, molecular branching degree, or film material composition as explained above with reference to possible polypropylene content, or also with reference to a reduced additive content, e.g. pigments and/or fillers, can be tailored in order to provide a film with the selected features. Alternatively or in combination process conditions may be selected, for example type of extrusion, e.g. cast or blown extrusion, cooling, stretching, in order to have the final sheet material for the wrapper material according to the present invention.

The individually packaged absorbent article according to the present invention guarantees a greatly increased discreetness to the user, as the selected wrapper material is much less noisy than current materials when manipulated, typically when the user breaks the seals to open the package, and then separates the absorbent article from the wrapper itself, in order to prepare the absorbent article for its intended use by exposing the adhesive element for attachment to the undergarment. Also the simple handling of the packaged product is quieter, providing the user with better self-confidence and tranquility. At the same time, the individually packaged absorbent article of the present invention can be manufactured with known high speed processes, with no need of drastic adjustments or changes.

Finally the individually packaged absorbent article of the present invention is also cheaper, as it uses a sensibly lighter material for the releasable wrapper.

Test Methods.

Tensile strength.

The tensile strength of the wrapper material of the individually packaged absorbent article of the present invention is evaluated according to the standard test method ASTM D 882-02 modified as specified below, and is the tensile strength corresponding to the maximum load. The following sections, unless otherwise indicated, replace the respective corresponding sections in the original ASTM D 882-02 test method.

Section 6.1 - The test specimens shall consist of strips of uniform width and thickness at least 20 mm longer than the grip separation used, or even slightly less, provided slippage relative to the grips is prevented as prescribed in Section 5.1.3.3.

Section 6.2 - The nominal width of the specimens shall be 25.4 mm.

Sections 9.2 and 9.3 - The speed of testing shall be set at 508 mm/min, which is optimized for polymeric films typically constituting the wrapper material according to the present invention. The initial strain rate is therefore not considered.

Section 10.2 - Measurement of the cross-sectional area of the specimen as a function of its width and thickness, as provided by original Section 10.2 of ASTM D 882-02, does not apply, as the tensile strength value is expressed as force per unit length, see Section 11.3 below.

Section 10.3 - Set the initial grip separation at 51 mm.

Section 10.4 - Set the rate of grip separation at 508 mm/min, see Section 9.2 above. Set a pre-load value at 0.075 N. Pre-load is meant to compensate for slack that may be present in the specimen when loaded by finding the first point at which the measured load (force) exceeds the input pre-load, and assigning an elongation value of zero at this point. Zero the calibrated load weighing, the extension indicator with the proviso above due to the set pre-load value, and the recording system.

Section 10.6 - In addition to the provisions of original Section 10.6, care shall be taken when placing the specimen to not exceed the selected pre-load value of 0.075 N. The instrument shall not be zeroed after the specimen has been placed.

Section 11.3 - Tensile Strength (nominal) shall be calculated by dividing the maximum load by the original width of the specimen (25.4 mm). The result shall be expressed in force per unit length, usually Newton per centimeter. This value shall be reported to two significant figures.

In general, the selected rate of grip separation and the initial distance between the grips are optimized for polymeric films typically constituting the wrapper material according to the present invention. In principle, if materials other than polymeric films are used, the provisions of original Sections 9.2, 9.3, 10.3, 10.4 of ASTM D 882-02 shall apply.

As also explained in the description above, the wrapper material for the individually packaged absorbent products of the present invention can be anisotropic, i.e. have different tensile strengths in machine direction and in cross direction. In this case, the provision of Section 6.6 of ASTM D 882-02 shall apply. For measurements of the wrapper material of an actual product, as already explained, different test specimens shall be actually prepared having their long axes respectively parallel to the major and to the minor axis of the wrapper material laid open flat on a plane surface, usually to the longer and shorter sizes thereof, for a typically rectangular wrapper material.

When the tensile strength of a wrapper material of an actual product has to be measured, the wrapper shall be opened carefully trying to not tear or strain the wrapper material, and laid flat on a plane surface. Alternatively, the longitudinal areas of the formed wrapper corresponding to the bag seals can be carefully cut, and the wrapper opened. The absorbent article has to be removed from the wrapper material, and care shall be taken not to deform the wrapper material in this operation. Specimens of the desired size can be then cut from the wrapper material along the direction parallel to the major axis of the material, and along the direction perpendicular thereto, in order to measure the tensile strength in both main directions of the wrapper material, according to what specified under Section 6.6 of the Test Method. The samples should be cut from the central portion of the wrapper material, trying to avoid the border areas interested by the bag seals. Such areas could be incorporated if needed in the end portions of the specimen meant to be held in the apparatus grips, but shall not be comprised in the free portion of the specimen (51.0 mm x 25.4 mm) comprised between the grips, where the actual measurement is made.

Coefficient of friction.

The coefficient of friction of the wrapper material of the individually packaged absorbent article of the present invention is evaluated as the kinetic coefficient of friction according to the standard test method ASTM D 1894-06. The Assembly of Apparatus of Fig. 1(c) has been adopted, selecting a speed setting for a crosshead motion of 127 mm/min, but any other suitable assembly and speed setting as prescribed in Section 7 of the standard method can be chosen.

Tear-propagation resistance.

The tear propagation resistance of the wrapper material of the individually packaged absorbent article of the present invention is evaluated according to the standard test method ASTM D 1938-06, with some modifications as explained below.

The die shape, referred to in Section 6.3 and Fig. 3 of the ASTM method, is changed such that the length is 100 mm and the width is 50 mm, while the length of the pre cut is kept at 50 mm. The two tongues A and B are now 25 mm wide, and since the grips should minimize both slippage and uneven stress distribution, as specified under Section 6.1.3, they should be typically at least as wide as the respective tongue width.

The grip separation speed, referred to in Section 9.2, is 300 mm/min; with reference to subsequent Section 9.3, the test is continued until the tear has propagated through 30 mm of the unslit portion, hence leaving a portion of the specimen still unslit.

As for Section 10.1, the average tear propagation force is calculated by averaging the load over a 30 mm interval, disregarding the initial portion of the curve.

The tear propagation resistance is considered to correspond to the average tear propagation force for wrapper materials having a load-time chart as that of Fig. 1 in the ASTM method, called "low-extensible films" therein. The tear propagation resistance is instead meant to correspond to the initial tear propagation force for wrapper materials having a load-time chart as that of Fig. 2 in the ASTM method, called "highly extensible films" therein. Wrapper materials according to the present invention can more likely have load-time charts characterized by Fig. 1.

In the context of the present invention the tear propagation resistance is intended with reference to a tear along a direction orthogonal to the longitudinal edge 44 of the longitudinal flap portion 42 of the wrapper 40, along which the sealing occurs, e.g. by crimping. Hence, as can be readily ascertained by the skilled person, specimens have to be cut from the raw material accordingly in order to match this orientation. When the tear propagation resistance of a wrapper material of an actual product has to be measured, the wrapper shall be opened carefully trying to not tear or strain the wrapper material, and laid flat on a plane surface. Alternatively, the longitudinal areas of the formed wrapper corresponding to the bag seals can be carefully cut, and the wrapper opened. The absorbent article has to be removed from the wrapper material, and care shall be taken not to deform the wrapper material in this operation. In particular, suitable rectangular specimens of the prescribed size can be cut from the central portion of the wrapper material, wherein the longer sides of the rectangular specimen are typically orthogonal to the longitudinal (longer) axis of the flattened out wrapper material. The samples should be cut from the central portion of the wrapper material, avoiding the border areas interested by the broken bag seals. Such areas could be incorporated if needed in the shorter end portions of the specimen, i.e. the one meant to stay free during the testing, or those of the two tongues which are to be held in the apparatus grips. Also areas where the wrapper material was folded in the formed pouch should be avoided when cutting the specimens.

Each dimension for which a value is defined herein is a technical dimension, which, in the context of the present invention is not to be understood literal. Hence, all embodiments having dimensions functionally equivalent to the dimensions stated herein are intended to be covered by the scope of the invention, e.g. a dimension of "40 mm" has to be understood as meaning "about 40 mm".

## Claims

1. An individually packaged absorbent article comprising an absorbent article having a body facing side, a garment facing side, two longitudinal sides and two transverse ends, said absorbent article having an adhesive element on said garment facing side, a releasable wrapper overlaying said garment facing side of said article and releasably affixed to said adhesive element, said absorbent article and said wrapper being folded as a unit about at least one fold-axis to define a package comprising said absorbent article, said individually packaged absorbent article **characterized in that** said releasable wrapper comprises a flexible sheet material having:
a basis weight of 5-19 g/m², and
a maximum tensile strength of at least 6.5 N/cm,
wherein said material of said releasable wrapper is selected among polymeric films having a thickness of 6-20 µ, and
wherein said film is a polyethylene film comprising at least 0.5% by weight, and less than 30% by weight polypropylene.

2. An individually packaged absorbent article according to claim 1, wherein said material of said releasable wrapper has a coefficient of friction, at least on the surface opposite to that in contact with said adhesive element, comprised between 0.1 and 0.7, evaluated as the kynetic coefficient of friction according to the standard test method ASTM D 1894-06.

3. An individually packaged absorbent article according to any preceding claim, wherein said flexible sheet material of said releasable wrapper comprises on the surface releasably affixed to said adhesive element a release agent.

4. An individually packaged absorbent article according to any preceding claim, wherein said absorbent article and said wrapper are folded as a unit about two transverse fold-axes.

5. An individually packaged absorbent article according to any preceding claim, wherein said flexible sheet material of said releasable wrapper has a tear propagation resistance of at least 0.18 N, evaluated according to the standard test method ASTM D 1938-06, with the modifications as described in the "Tear-propagation resistance" section of the application.

## Patentansprüche

1. Einzeln verpackter Absorptionsartikel, umfassend einen Absorptionsartikel mit einer körperseitigen Seite, einer bekleidungsseitigen Seite, zwei längsverlaufenden Seiten und zwei querverlaufenden Enden, wobei der Absorptionsartikel ein Klebeelement auf der bekleidungsseitigen Seite aufweist, wobei eine abziehbare Einwickelfolie die bekleidungsseitige Seite des Absotptionsartikels überdeckt und an dem Klebeelement lösbar befestigt ist, wobei der Absorptionsartikel und die Einwickelfolie als eine Einheit um mindestens eine Faltachse gefaltet sind, um eine Verpackung zu definieren, die den Absorptionsartikel umfasst, wobei der einzeln verpackte Absorptionsartikel **dadurch gekennzeichnet ist, dass** die abziehbare Einwickelfolie ein flexibles Schichtmaterial umfasst, das Folgendes aufweist:
ein Basisgewicht von 5 bis 19 g/m², und
eine maximale Zugfestigkeit von mindestens 6,5 N/cm,
wobei das Material der abziehbaren Einwickelfolie aus Polymerfolien mit einer Dicke von 6 bis 20 µ ausgewählt ist, und
wobei die Folie eine Polyethylenfolie ist, die mindestens 0,5 Gew.-% und
weniger als 30 Gew.-% Polypropylen umfasst.

2. Einzeln verpackter Absorptionsartikel nach Anspruch 1, wobei das Material der abziehbaren Einwickelfolie einen Reibungskoeffizienten mindestens auf der Oberfläche gegenüber derjenigen, die mit dem Klebeelement in Kontakt steht, zwischen 0, und 0,7 umfasst, der gemäß dem Standardprüfverfahren ASTM D 1894-06 als der kinetische Reibungskoeffizient bewertet wird.

3. Einzeln verpackter Absorptionsartikel nach einem der vorstehenden Ansprüche, wobei das flexible Schichtmaterial der abziehbaren Einwickelfolie auf der Oberfläche, die an dem Klebeelement lösbar befestigt ist, ein Trennmittel umfasst.

4. Einzeln verpackter Absorptionsartiket nach einem der vorstehenden Ansprüche, wobei der Absorptionsartikel und die Einwickelfolie als eine Einheit um zwei querverlaufende Faltachsen gefaltet sind.

5. Einzeln verpackter Absorptionsartikel nach einem der vorstehenden Ansprüche, wobei das flexible Schichtmaterial der abziehbaren Einwickelfolie eine Weiterreißfestigkeit von mindestens 0,18 N aufweist, die gemäß dem Standardprüfverfahren ASTM D 1938-06 mit den in dem Abschnitt "Weiterreißfestigkeit" der Anmeldung beschriebenen Modifikationen bewertet wird,

## Revendications

1. Article absorbant conditionné individuellement comprenant un article absorbant ayant un côté faisant face au corps, un côté faisant face au vêtement, deux côtés longitudinaux et deux extrémités transversales, ledit article absorbant ayant un élément adhésif sur ledit côté faisant face au vêtement, un emballage libérable se trouvant au-dessus dudit côté faisant face au vêtement dudit article et fixé de manière libérable audit élément adhésif, ledit article absorbant et ledit emballage étant plié en tant qu'unité autour d'au moins un axe de pliage pour définir un conditionnement comprenant ledit article absorbant, ledit article absorbant conditionné individuellement **caractérisé en ce que** ledit emballage libérable comprend un matériau en feuille souple ayant :
une masse surfacique de 5 à 19 g/m², et
une résistance maximale à la traction d'au moins 6,5 N/cm,
dans lequel ledit matériau dudit emballage libérable est choisi parmi des films polymères ayant une épaisseur de 6 à 20 µ, et
dans lequel ledit film est un film de polyéthylène comprenant au moins 0,5 % en poids, et moins de 30 % en poids de polypropylène.

2. Article absorbant conditionné individuellement selon la revendication 1, dans lequel ledit matériau dudit emballage libérable a un coefficient de flottement, au moins sur la surface opposée à celle en contact avec ledit élément adhésif, compris entre 0,1 et 0,7, évalué en tant que coefficient de frottement cinétique selon le procédé de test normalisé ASTM D 1894-06.

3. Article absorbant conditionné individuellement selon l'une quelconque des revendications précédentes, dans lequel ledit matériau en feuille souple dudit emballage libérable comprend sur la surface fixée de manière libérable audit élément adhésif un agent anti-adhésif.

4. Article absorbant conditionné individuellement selon l'une quelconque des revendications précédentes, où ledit article absorbant et ledit emballage sont pliés en tant qu'unité autour de deux axes de pliage transversaux.

5. Article absorbant conditionné individuellement selon l'une quelconque des revendications précédentes, dans lequel ledit matériau en feuille souple dudit emballage libérable a une résistance à l'allongement d'une déchirure d'au moins 0,18 N, évaluée selon le procédé de test normalisé ASTM D 1938-06, avec les modifications telles que décrites dans la section « résistance à l'allongement de déchirure » de la demande.
